(19) Europäisches Patentamt
European Patent Office
Office européen des brevets

(11) Veröffentlichungsnummer: **0 055 817**
**B1**

(12) # EUROPÄISCHE PATENTSCHRIFT

(45) Veröffentlichungstag der Patentschrift: **13.08.86**

(51) Int. Cl.⁴: **A 61 K 9/00**

(21) Anmeldenummer: **81109220.4**

(22) Anmeldetag: **29.10.81**

(54) **Stabile injizierbare beta-Carotin-Solubilisate und Verfahren zu ihrer Herstellung.**

(30) Priorität: **19.12.80 DE 3048000**

(43) Veröffentlichungstag der Anmeldung: **14.07.82 Patentblatt 82/28**

(45) Bekanntmachung des Hinweises auf die Patenterteilung: **13.08.86 Patentblatt 86/33**

(84) Benannte Vertragsstaaten: **BE CH DE FR GB LI NL**

(56) Entgegenhaltungen:
**DE-A-2 236 899**
**DE-A-3 048 000**
**DE-B-1 210 127**
**DE-C-970 772**
**US-A-2 417 299**
**US-A-2 518 230**
**US-A-2 524 247**
**US-A-4 075 333**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Hoppe, Peter Paul, Dr., In der Dreispitz 7, D-6706 Wachenheim (DE)**
Erfinder: **Schneider, Joachim U., Dr., Plauserstrasse 1, D-6719 Weisenheim (DE)**
Erfinder: **Schulz, Bernhard, Dr., Kurpfalzring 28, D-6830 Schwetzingen (DE)**
Erfinder: **Tiefenbacher, Hubert, Dr., Hausaeckerstrasse 49, D-7022 Leinfelden-Echterdingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 055 817 B1

LIBER, STOCKHOLM 1986

**Beschreibung**

Die Erfindung betrifft stabile injizierbare Solubilisate von β-Carotin sowie ein Verfahren zu ihrer Herstellung.

β-Carotin hat die chemische Formel:

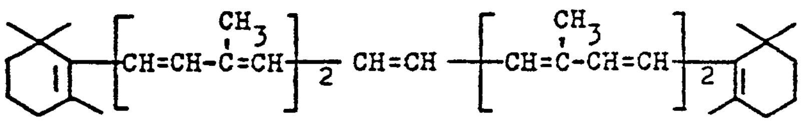

β-Carotinmangel beim Rind bedingt nach den Untersuchungen von Lotthammer (vgl. Dtsch. Tierärztl. Wschr. Nr. *82* (1975) S. 444—49; Nr. *83* (1976) S. 353—58; Nr. *84* (1977) S. 220—26 und S. 307—310; Nr. *85* (1978) S. 7—12) einen gestörten Brunstablauf mit schlechter Fruchtbarkeit. Kennzeichen des Mangels ist ein niedriger β-Carotin-Blutspiegel.

Bislang wird β-Carotin-Mangel durch Fütterung von carotinreichen Einzelfuttermitteln wie Luzernegrünmehl, Grasgrünmehl oder Karotten und/oder synthetischem β-Carotin-Trockenpulver behandelt. Bei oraler Verabreichung von β-Carotin ist jedoch der Einfluß auf den β-Carotin-Blutspiegel gering, weil β-Carotin aus dem Darmtrakt schlecht resorbiert wird.

Dagegen kann durch parenterale Verabreichung von β-Carotin der Blutspiegel schnell und über einen längeren Zeitraum erhöht werden.

Dazu müssen die zu injizierenden Zubereitungen in möglichst fein verteilter Form einen relativ hohen β-Carotin-Gehalt aufweisen, damit das einzuspritzende Volumen möglichst klein ist.

Bei Tieren, die einen niederen β-Carotin-Spiegel im Blutplasma aufweisen, ist eine schnelle (innerhalb zwei Tagen) und ökonomische Anhebung des β-Carotin-Spiegels auf gastroenteralem Wege nicht möglich. Das β-Carotin muß parenteral verabreicht werden.

Übliche β-Carotin-Lösungen in Pflanzenölen weisen jedoch höchstens einen Gehalt von 0,3 bis 0,5% auf (Ullmanns Enzyklopädie der technischen Chemie, Band 11 (1976), Seite 106). Solche Konzentrationen sind aber zu niedrig, um in einem gebräuchlichen Injektionsvolumen von 10—20 ml die erforderliche Dosis von 500 mg β-Carotin zu verabreichen.

Aus der Japanischen Patentveröffentlichung Nr. 38 556/1970 ist es bekannt, Carotinoid-Verbindungen durch gemeinsames Lösen mit aliphatischen Estern von Zucker, thermisches Behandeln und Entfernen des Lösungsmittels in wasserlösliche Carotinoidpräparate zu überführen. Auf diese Weise können jedoch nur etwa 0,2%ige Lösungen von β-Carotin hergestellt werden.

Aus der DE—C—970 772, der US—A—2 417 299, der US—A—2 518 230 und der US—A—2 524 247 ist die Herstellung von wäßrigen Lösungen von lipophilen Vitaminen, insbesondere von den Vitaminen A, D, E und $K_1$ mit Hilfe von nicht-ionogenen Emulgatoren bekannt. Als nicht-ionogene Emulgatoren werden im wesentlichen Polyoxyethylenether mit langkettigen aliphatischen Alkoholen oder Polyoxyethylenderivate von partiell mit langkettigen Fettsäuren veresterten mehrwertigen Alkoholen verwendet.

Wie aus der DE—B—1 210 127 hervorgeht, sind solche mit nicht-ionogenen Emulgatoren hergestellten Lösungen in zahlreichen Fällen nur kurzzeitig oder überhaupt nicht verwendbar, da sie nicht über die notwendige physikalische Stabilität verfügen. Es werden daher Zusätze von ionenaktiven Netzmitteln als Stabilisatoren verwendet. Gemäß dem Verfahren dieser DE—B—1 210 127 erhaltene wäßrige β-Carotin-Solubilisate, die polyoxyethyliertes Ricinusöl oder Polyoxyethylen-Sorbitan-Fettsäureester als Emulgatoren sowie ionenaktive Netzmittel enthalten, sind jedoch nicht längere Zeit stabil, da das Carotin auskristallisiert.

Deshalb wurde gemäß DE—OS 22 36 899 schon vorgeschlagen, durch Verwendung spezieller Seifen, nämlich der Seifen von Tris-(hydroxymethyl)-aminomethan mit gesättigten oder ungesättigten Fettsäuren mit 9 bis 20 Kohlenstoffatomen höher konzentrierte Emulsionen herzustellen. Die dort vorgeschlagene Methode befriedigt aber noch nicht, da einerseits die Durchführung in der Praxis Schwierigkeiten bereitet und andererseits nur mit Wasser mischbare Carotinoid-Emulsionen hergestellt werden können, die maximal 4% β-Carotin enthalten.

Es war daher Aufgabe der Erfindung, höher konzentrierte klare stabile Emulsionen bzw. Solubilisate des β-Carotins herzustellen.

Gegenstand der Erfindung sind ein Verfahren zur Herstellung von β-Carotin-Solubilisaten, das dadurch gekennzeichnet ist, daß man in einen auf 160 bis 180°C erhitzten, nicht-ionogenen, zur Herstellung von Solubilisaten geeigneten Emulgator β-Carotin in Mengen von insgesamt 20 bis 30 Gew.%, bezogen auf den Emulgator, einträgt, die heiße homogene Mischung durch Zugabe von Wasser schnell auf Temperaturen unter 100°C abkühlt und durch weitere Wasserzugabe die Zubereitung auf die gewünschte Konzentration von 3 bis 6 Gew.% einstellt, sowie die durch dieses Verfahren erhältlichen stabilen injizierbaren β-Carotin-Solubilisate.

Es war überraschend, daß auf die beschriebene Weise nicht nur eine milchig trübe β-Carotin-Emulsion, sondern ein durchsichtiges β-Carotin-Solubilisat mit einem β-Carotin-Gehalt von bis zu 6 Gew.% erhalten werden kann.

Als nicht-ionogene, zur Herstellung von Solubilisaten geeignete Emulgatoren kommen solche mit einem HLB-Wert (vgl. H. P. Fiedler, Lexikon der Pharmazie, Kosmetik und angrenzenden Gebiete, 1971, Seiten 263—270, besonders Seiten 267—69), von 12 bis 16 in Betracht, insbesondere ethoxylierte Triglyceride von Fettsäuren mit 12 bis 18 Kohlenstoffatomen, die 20 bis 60 Oxethyleneinheiten enthalten; ethoxylierte Sorbitanfettsäureester mit etwa 20 Oxethyleneinheiten oder ethoxylierte Monohydroxifettsäuren mit 14 bis 17 Oxethyleneinheiten, wie sie in der DE—OS 29 11 241 beschrieben sind. Derartige Emulgatoren heißen auch Solubilisatoren, weil sie sich in Wasser lösen und dadurch als Lösungsvermittler für lipophile Substanzen wirken, indem diese in micellarer Lösung gehalten werden. Micellare Lösungen zeichnen sich durch Transparenz und Klarheit aus.

Als besonders geeignete nicht-ionogene Emulgatoren seien beispielsweise genannt:

Glycerinpolyoxethylenglykolricinoleat, Glycerinpolyoxethylenglykoloxystearat, Polyoxethylen(20)sorbitanmonooleat, Polyoxethylen(20)sorbitanmonostearat und Monohydroxystearinsäure mit 15 Oxethyleneinheiten.

Bei der Herstellung der Solubilisate geht man im einzelnen so vor, daß man in den auf 160 bis 180°C erwärmten Emulgator, der übliche Antioxydantien enthalten kann, 20 bis 30 Gew.% an β-Carotin, bezogen auf den Emulgator, portionsweise zugibt, wobei das β-Carotin schmilzt und sich praktisch sofort löst. Durch Zugabe von Wasser senkt man die Temperatur der Mischung rasch auf unter 100°C. Anschließend stellt man durch weitere Wasserzugabe die Zubereitung auf die gewünschte Konzentration ein. Nach Filtration und Abkühlen auf Raumtemperatur erhält man ein stabiles 4- bis 6gew.%iges, vorzugsweise ein etwa 5%iges, transparentes Solubilisat das noch nach 12 Monaten stabil ist.

Als übliche Antioxydantien, die bei dem erfindungsgemäßen Verfahren mitverwendet werden können, seien beispielsweise genannt: Butylhydroxitoluol, Butylhydroxianisol und d,l-α-Tocopherol. Die Antioxydantien verwendet man im allgemeinen in Mengen von 10 bis 20 Gew.%, bezogen auf eingesetztes β-Carotin.

Gemäß den Angaben des Beispiels der DE—OS 22 36 899 soll bei Einbringen des β-Carotins in den Emulgator die Temperatur möglichst 110°C nicht übersteigen. Diese Forderung ist für den Fachmann verständlich, da bei höheren Temperaturen eine Isomerisierung des β-Carotins zu befürchten ist.

Es war daher überraschend, daß bei der erfindungsgemäßen Arbeitsweise ein für Injektionszwecke ausreichend konzentriertes Solubilisat erhalten wird, das im Tier die volle Wirkung entfaltet, obgleich ein Teil des β-Carotins vermutlich isomerisiert.

## Beispiel

In einem 2-1-4-Hals-Rundkolben mit Rührer, Stickstoffeinlaß und Rückflußkühler werden 300 g Cremophor EL® (Glycerinpolyoxethylenglykolricinoleat) und 6 g Butylhydroxitoluol eingebracht. Unter Rühren und Stickstoffbegasung wird das Gemisch erwärmt bis eine Innentemperatur von 160°C erreicht ist. Innerhalb von ca. 5 Minuten werden 66 g β-Carotin hinzugegeben. Man entfernt das Heizbad und gibt tropfenweise Wasser hinzu bis die Innentemperatur des Gemisches auf 100°C abgesunken ist. Durch rasche Zugabe der restlichen Menge Wasser (Gesamtmenge 828 g) und Erwärmen mit dem Heizbad wird eine Temperatur von 60 bis 80°C eingestellt. Man filtriert das noch warme Solubilisat durch eine Glasfilterfritte und erhält eine ca. 5%ige transparente β-Carotinlösung, die als Injektionslösung geeignet ist.

## Tierversuche

3 Versuchsgruppen (schwarzbunte Färsen) wurden miteinander verglichen.

Die Versuchsgruppen wurden wie folgt behandelt:

Gruppe 1 erhielt intramuskulär 10 ml eines erfindungsgemäßen wäßrigen β-Carotin-Solubilisats enthaltend 450 mg β-Carotin.

Gruppe 2 erhielt eine intramuskuläre Injektion einer öligen β-Carotin-Lösung enthaltend 450 mg β-Carotin.

Gruppe 3 erhielt keine β-Carotin-Injektion.

Gemessen wurde der durchschnittliche β-Carotin-Plasmaspiegel (μg/100 ml) der Versuchstiere vor der Behandlung sowie 1, 2, 4 und 32 Tage nach der Behandlung.

| Gruppe | Durchschnittlicher β-Carotin-Plasmaspiegel (µg/100 ml) Tag 0 | Tag 1 | Tag 2 | Tag 4 | Tag 32 |
|---|---|---|---|---|---|
| 1 | 230 | 1 547 | 1 705 | 1 214 | 369 |
| 2 | 221 | 288 | 371 | 355 | 232 |
| 3 | 182 | 183 | 191 | 204 | 207 |

Wie aus dem Versuch hervorgeht, lag bei Verabreichung des wäßrigen β-Carotin-Injektionspräparates der Blutspiegel zu jedem Zeitpunkt signifikant höher als beim Vergleichsprodukt. Somit ist es möglich, den β-Carotinmangel mit einer einmaligen Injektion für mehr als 1 Monat zu beseitigen und einen physiologischen Brunstablauf zu gewährleisten.

**Patentansprüche**

1. Verfahren zur Herstellung von β-Carotin-Solubilisaten, dadurch gekennzeichnet, daß man in einen auf 160 bis 180°C erhitzten, nicht-ionogenen, zur Herstellung von Solubilisaten geeigneten Emulgator, β-Carotin in Mengen von insgesamt 20 bis 30 Gew.%, bezogen auf den Emulgator, einträgt, die heiße homogene Mischung durch Zugabe von Wasser schnell auf Temperaturen unter 100°C abkühlt und durch weitere Wasserzugabe die Zubereitung auf die gewünschte Konzentration von 3 bis 6 Gew.% einstellt.

2. Verfahren gemäß Anspruch 1, dadurch gekennzeichnet, daß man als nicht-ionogenen Emulgator einen solchen verwendet, der einen HBL-Wert von 12 bis 16 aufweist.

3. Verfahren gemäß Anspruch 1, dadurch gekennzeichnet, daß man als nicht-ionogenen Emulgator ethoxylierte Triglyceride von Fettsäuren mit 12 bis 18 C-Atomen, die 20 bis 60 Oxethyleneinheiten enthalten, verwendet.

4. Verfahren gemäß Anspruch 1, dadurch gekennzeichnet, daß man als nicht-ionogenen Emulgator Glycerinpolyoxethylenglykolricinoleat verwendet.

5. Verfahren gemäß Anspruch 1, dadurch gekennzeichnet, daß man als nicht-ionogenen Emulgator Glycerinpolyoxethylenglykoloxistearat verwendet.

6. Verfahren gemäß Anspruch 1, dadurch gekennzeichnet, daß man als nicht-ionogenen Emulgator polyoxethylierte Sorbitanfettsäureester verwendet.

7. Verfahren gemäß Anspruch 1, dadurch gekennzeichnet, daß man als nicht-ionogenen Emulgator Polyoxethylen(20)sorbitanmonooleat verwendet.

8. Verfahren gemäß Anspruch 1, dadurch gekennzeichnet, daß man als nicht-ionogenen Emulgator Polyoxyethylen(20)sorbitanmonostearat verwendet.

9. Verfahren gemäß Anspruch 1, dadurch gekennzeichnet, daß man als nicht-ionogenen Emulgator Monohydroxistearinsäure mit 15 Oxethyleneinheiten verwendet.

10. Verfahren gemäß den Ansprüchen 1 bis 9, dadurch gekennzeichnet, daß man hierbei ein Antioxidans mitverwendet.

11. Verfahren gemäß Anspruch 1, dadurch gekennzeichnet, daß man als Antioxidans Butylhydroxitoluol, Butylhydroxianisol, d,l,α-Tocopherol in Mengen von 10 bis 20 Gew.-%, bezogen auf β-Carotin, verwendet.

12. Stabile wäßrige Injektionslösungen mit einem β-Carotin-Gehalt von 3 bis 6 Gew.-%, erhältlich durch Eintragen von β-Carotin in einen auf 160 bis 180°C erhitzten, nicht-ionogenen, zur Herstellung von Solubilisaten geeigneten Emulgator in Mengen von insgesamt 20 bis 30 Gew.-% β-Carotin, bezogen auf den Emulgator, schnelles Abkühlen der erhaltenen heißen homogenen Mischung durch Zugabe von Wasser auf Temperaturen von unter 100°C und weitere Wasserzugabe zur Einstellung der Zubereitung auf die gewünschte Endkonzentration von 3 bis 6 Gew.-%.

**Revendications**

1. Procédé de préparation de solutés de bêta-carotène, caractérisé en ce que l'on introduit dans un émulsionnant non ionogène, pouvant être utilisé pour la préparation de solutés et chauffé entre 160 et 180°C, du bêta-carotène en une proportion comprise entre 20 et 30% en poids par rapport à l'émulsionnant, on refroidit le mélange homogène chaud rapidement, par addition d'eau, à une température inférieure à 100°C, puis on amène la formulation par l'addition d'une quantité supplémentaire d'eau à la concentration voulue de 3 à 6% en poids.

2. Procédé suivant la revendication 1, caractérisé en ce que l'émulsionnant non ionogène est un émulsionnant possédant un équilibre hydro-lipophile compris entre 12 et 16.

3. Procédé suivant la revendication 1, caractérisé en ce que l'émulsionnant non ionogène est choisi parmi les triglycérides éthoxylés d'acides gras en $C_{12}$ à $C_{18}$, contenant 20 à 60 unités oxyde d'éthylène.

4. Procédé suivant la revendication 1, caractérisé en ce que l'émulsionnant non ionogène est un ricinoléate de glycérine-polyoxyéthylène-glycol.

5. Procédé suivant la revendication 1, caractérisé en ce que l'émulsionnant non ionogène est un oxystéarate de glycérine-polyoxyéthylène-glycol.

6. Procédé suivant la revendication 1, caractérisé en ce que l'émulsionnant non ionogène est un ester d'acide gras de sorbitanne polyéthoxylé.

7. Procédé suivant la revendication 1, caractérisé en ce que l'émulsionnant non ionogène est du monooléate de polyoxyéthylène(20)-sorbitanne.

8. Procédé suivant la revendication 1, caractérisé en ce que l'émulsionnant non ionogène est du monostéarate de polyoxyéthylène(20)-sorbitanne.

9. Procédé suivant la revendication 1, caractérisé en ce que l'émulsionnant non ionogène est de l'acide monohydroxy-stéarique comprenant 15 unités oxyde d'éthylène.

10. Procédé suivant les revendications 1 à 9, caractérisé en ce que l'on utilise en outre un anti-oxygène.

11. Procédé suivant la revendication 1 (lire 10), caractérisé en ce que l'anti-oxygène est choisi parmi l'hydroxy-toluène butylique, l'hydroxy-anisole butylique et le d,l-α-tocophérol, utilisé à raison de 10 à 20% en poids par rapport au β-carotène.

12. Solutions aqueuses stables, pouvant être injectées, d'une teneur en bêta-carotène de 3 à 6% en poids, obtenues par l'introduction de bêta-carotène dans un émulsionnant non ionogène, convenant pour la préparation de solutés et chauffé entre 160 et 180°C, à raison de 20 à 30% en poids de bêta-carotène par rapport à l'émulsionnant, refroidissement rapide du mélange homogène chaud formé par addition d'eau à une température inférieure à 100°C et réglage de la teneur finale désirée en bêta-carotène de la formulation par addition d'eau supplémentaire entre 3 et 6% en poids.

**Claims**

1. A process for the preparation of a β-carotene micellar solution, wherein a total of from 20 to 30% by weight, based on the emulsifier, of β-carotene is introduced into a non-ionic emulsifier which is suitable for the preparation of micellar solutions and has been heated to from 160 to 180°C, the hot homogeneous mixture is cooled rapidly to below 100°C by adding water, and the formulation is brought to the desired concentration of from 3 to 6% by weight by adding further water.

2. A process as claimed in claim 1, wherein a non-ionic emulsifier having an HLB value of from 12 to 16 is used.

3. A process as claimed in claim 1, wherein an oxyethylated triglyceride of a fatty acid containing 12 to 18 carbon atoms and 20—60 oxyethylene units is used as the non-ionic emulsifier.

4. A process as claimed in claim 1, wherein glycerol polyoxyethylene glycol ricinoleate is used as the non-ionic emulsifier.

5. A process as claimed in claim 1, wherein glycerol polyoxyethylene glycol hydroxystearate is used as the non-ionic emulsifier.

6. A process as claimed in claim 1, wherein a polyoxyethylated sorbitan fatty acid ester is used as the non-ionic emulsifier.

7. A process as claimed in claim 1, wherein polyoxyethylene-20 sorbitan monooleate is used as the non-ionic emulsifier.

8. A process as claimed in claim 1, wherein polyoxyethylene-20 sorbitan monostearate is used as the non-ionic emulsifier.

9. A process as claimed in claim 1, wherein the adduct of monohydroxystearic acid containing 15 ethylene oxide units is used as the non-ionic emulsifier.

10. A process as claimed in claims 1 to 9, wherein an antioxidant is co-used.

11. A process as claimed in claim 1, wherein from 10 to 20% by weight, based on the β-carotene, of butylhydroxytoluene, buthylhydroxyanisole or d,l-α-tocopherol is used as the antioxidant.

12. A stable aqueous injection solution which contains from 3 to 6% by weight of β-carotene and can be obtained by introducing a total of from 20 to 30% by weight, based on the emulsifier, of β-carotene into a non-ionic emulsifier, which is suitable for the preparation of a micellar solution and has been heated to from 160 to 180°C, rapidly cooling the resulting hot homogeneous mixture to below 100°C by adding water, and bringing the formulation to the desired final concentration of from 3 to 6% by weight by adding further water.